# EUROPÄISCHE PATENTANMELDUNG

(11) **EP 2 149 390 A2**
(43) Veröffentlichungstag der Anmeldung: **03.02.2010**
(21) Anmeldenummer: 09163984.9
(22) Anmeldetag: 29.06.2009
(51) Int. Cl.: A61N 1/05

(54) **Implantierbare Katheter- oder Elektrodenleitung**

(30) Priorität: 28.07.2008 DE 102008040773
(71) Anmelder: Biotronik CRM Patent AG, 6341 Baar (CH)
(72) Erfinder: Flach, Erhard, 12305, Berlin (DE); Landgraf, Tassilo, 12277, Berlin (DE); Doce, Enlen, 13347, Berlin (DE)
(74) Vertreter: Lindner-Vogt, Karin L.

(57) **Zusammenfassung**

Implantierbare Katheter- oder Elektrodenleitung mit einem langgestreckten biegsamen Leitungskörper und mit dem Leitungskörper verbundenen Fixierungsmitteln zur Fixierung der Katheter- oder Elektrodenleitung in vorbestimmter Lage in einem Gefäß oder einer Körperhöhlung eines Patienten, wobei zwischen dem Leitungskörper und den Fixierungsmitteln eine lösbare Verbindung vorgesehen ist derart, dass nach Lösen der Verbindung eine Explantation der Katheter- oder Elektrodenleitung unter Verbleib der Fixierungsmittel im Körper des Patienten möglich ist.

## Beschreibung

Die Erfindung betrifft eine Implantierbare Katheter- oder Elektrodenleitung mit einem langgestreckten biegsamen Leitungskörper und mit dem Leitungskörper verbundenen Fixierungsmitteln zur Fixierung der Leitung in vorbestimmter Lage in einem Gefäß oder einer Körperhöhlung eines Patienten.

Hierbei wird unter einer Katheter- oder Elektrodenleitung im allgemeinen Sinne jedes im Körper eines Menschen oder Tieres zu fixierende Katheter oder jedwede Art von Elektrodenleitung zur mindestens temporären intrakorporalen Fixierung verstanden, also etwa Katheter zum Zuführen von Fluiden oder Gegenständen in Körperhöhlungen oder Gefäße oder zur Abführung von Flüssigkeiten aus solchen, Schrittmacherelektrodenleitungen, Schockelektrodenleitungen, Gefäßstimulationsleitungen etc.

Derartige Katheter- oder Elektrodenleitungen sind in unübersehbarer Vielgestaltigkeit seit langem bekannt und auch als verschiedenartigste Produkte im Handel. Speziell im Verlaufe der Entwicklung der Technik der Schrittmacher und Kardioverter wurde besonderes Augenmerk auf eine sichere Fixierung von Elektrodenleitungen direkt am zu stimulierenden oder zu defibrillierenden Herzgewebe gelegt, und es wurden diverse Fixierungsmechanismen zur Erfüllung dieses Zwecks entwickelt.

Gefäß-Elektroden wie Coronar Sinus Elektroden müssen sehr dünn und flexibel sein, um in die gewünschten Zielvenen des Herzen implantiert werden zu können. Diese Elektroden müssen in den Zielgebieten sicher verankert werden. Darüber hinaus wird von klinischer Seite gewünscht, dass die Elektroden später wieder explantierbar sind. Passive Fixierungen wie z.B. ein distales Gewinde, eine Helix oder andere Vorformungen des E-lektrodenkörpers führen nach wie vor zu Dislokationsraten von 10% - 15%. Vorteilhaft bei diesen Elek-troden ist die spätere komplikationsarme Explantationsmöglichkeit. Aktiv fixierbare Elektroden mit einem distalen Spreizelement bieten zwar eine gute akute Fixierung, erzeugen aber durch Ein- und Umwachsen des Spreizelements einen intensiven Formschluss der Elektrode zur Venenwand. Damit sind sie i.d.R. nicht mehr explantierbar.

Der Erfindung liegt daher die Aufgabe zugrunde, eine verbesserte Katheter- bzw. Elektrodenleitung bereitzustellen, die einerseits bei der Implantation schnell und sicher fixiert werden kann und andererseits auch wieder ohne größere Mühe und erneute Traumatisierung explantierbar sein soll.

Diese Aufgabe wird durch eine Katheter- oder Elektrodenleitung mit den Merkmalen des Anspruchs 1 bzw. des Anspruchs 11 gelöst. Zweckmäßige Fortbildungen des Erfindungsgedankens sind Gegenstand der abhängigen Ansprüche.

Die Erfindung geht von dem wesentlichen Gedanken aus, dass die Elektrode nach einer bestimmten Zeitdauer, in der eine Sicherheit gegen Dislokation durch umwachsendes Gewebe noch nicht gegeben sein kann und leitungseigene Fixierungsmittel diese Sicherheit bieten müssen, mit den Fixierungsmitteln nicht mehr fest verbunden ist bzw. die Fixierungsmittel mindestens in einem Maße zerstört sind, dass sie ihre Fixierungswirkung hinsichtlich der Elek-trodenleitung weitgehend verloren haben. Die Elektrodenleitung kann dann, ohne Traumatisierung durch ein "Herausreißen" der Fixierungsmittel, vergleichsweise leicht wieder aus dem Gefäß oder Körperhohlraum herausgezogen werden. Mithin sieht die Erfindung in ihrer ersten Ausprägung eine lösbare Verbindung vor derart, dass nach Lösen der Verbindung eine Explantation der Katheter- oder Elektrodenleitung unter Verbleib der Fixierungsmittel im Körper des Patienten möglich ist. In der zweiten Ausprägung der Erfindung sind resorbierbare Schaumstoff- oder Vlieselemente als Fixierungsmittel vorgesehen, die sich im Körper auflösen, wonach ebenfalls eine leichte Explantation der Leitung möglich ist.

In einer Ausführung der Erfindung weist die Verbindung ein resorbierbares oder sich selbsttätig zersetzendes Verbindungselement auf. Hierbei können Material und Dimensionen des Verbindungselementes derart gewählt sein, dass die Resorption oder Zersetzung nach einer vorbestimmten Zeitspanne zum Lösen der Verbindung führt, innerhalb derer die Katheter- oder Elektrodenleitung in einem solchen Maße von Körpergewebe umwachsen ist, dass sie nicht mehr selbsttätig disloziert. Das gleiche Materialauswahl- bzw. Dimensionierungsprinzip gilt für jene Ausprägung des Erfindungsgedankens, in der die Fixierungsmittel als solche resorbier- bzw. zersetzbar ausgebildet sind.

Geeignete resorbierbare oder sich unter dem Einfluss von Körperflüssigkeiten zersetzende Materialien sind etwa als Nahtmaterial aus der Chirurgie und aus der Gelenk- und Knochenprothetik seit längerem bekannt und können grundsätzlich auch im Rahmen der Erfindung eingesetzt werden. Dazu gehören etwa resorbierbare Kunststoffe oder auch bestimmte Metalle und deren Legierungen, wie etwa Magnesium und Mg-Legierungen.

Alternativ zur vorgenannten Ausführung kann vorgesehen sein, dass die Verbindung ein durch Energieeintrag in die Leitung, insbesondere durch Anlegen eines elektrischen Stromes oder Einleitung von UV-Strahlung, Licht oder IR-Strahlung, zerstörbares Verbindungselement aufweist. Die Ausführung der Katheter- bzw. Elektrodenleitung mit lichtleitenden Eigenschaften, etwa durch Einbettung von Lichtwellenleitern, ermöglicht eine gezielte Applikation der erwähnten Strahlung im Bereich der Anbringung der Fixierungsmittel und die Zerstörung der Verbindung mit dem Leitungskörper etwa durch einen UV-Strahlungsimpuls oder eine milde Erhitzung mittels IR-Strahlung. Speziell bei einer Elektrodenleitung, die ohnedies eine Leitungsverbindung zwischen dem proximalen Ende und am oder nahe dem distalen Ende angeordneten Elektroden enthält, lässt sich der Verbindungsstelle zwischen Fixierungsmitteln und Leitungskörper auch eine Spannung oder eine Folge von Spannungspulsen zuführen, die das entsprechende Verbindungsmittel auf elektrochemischem Wege zerstört.

In einer besonders einfachen Ausführung ist vorgesehen, dass die Verbindung einen resorbier- oder zerstörbaren Haltering aufweist, der die Fixierungsmittel nur in seinem intakten Ausgangszustand auf dem Leitungskörper festhält. Diese Ausführung ist besonders einfach und kostengünstig in der Herstellung, denn zunächst können geeignet geformte Fixierungsmittel einfach auf den Leitungskörper aufgeschoben werden, auf diese wird dann wiederum der Haltering aufgesteckt, und dieser presst sie auf den Leitungskörper, solange er seine Integrität behält. Es versteht sich, dass dieser Kraftschluss durch eine formschlüssige Verbindung zwischen Leitungskörper und Fixierungsmitteln unterstützt oder insgesamt durch einen Formschluss zwischen Leitungskörper, Fixierungsmitteln und Haltering ersetzt sein kann.

Eine weitere spezielle Ausführung sieht vor, dass die Fixierungsmittel auf separaten Ringsegmenten angeordnet sind, die den Leitungskörper jeweils nur abschnittsweise (z. B. als Halbring zur Hälfte) umschließen und vom Verbindungselement auf dem Leitungskörper festgehalten werden, nach Resorption oder Zerstörung des Verbindungselementes sich aber vom Leitungskörper lösen (lassen).

Eine ähnliche, besonders leicht herzustellende und handhabende Ausführung zeichnet sich durch ein federelastisches Fixierungselement aus, das in eine annähernd geschlossene Ringform zusammen gedrückt werden und in diesem Zustand einen Leitungskörper dicht umschließen kann, bei Fortfall einer radial von außen wirkenden Haltekraft aber in eine annähernde Kreissegmentform aufspringt und sich hierdurch vom Leitungskörper löst. Zur Herstellung eines solchen Fixierungselementes eignet sich eine federelastische Metalllegierung (z.B. Nitinol) oder auch ein entsprechendes Kunststoffmaterial, ein sogenanntes Gedächtnismetall o. ä..

In jener Ausprägung der Erfindung, bei der als Fixierungsmittel Schaumstoff- oder Vlieselemente vorgesehen sind, sind diese optional aus einem in Blut quellfähigem Material gebildet, wodurch nach der Implantation eine Volumenvergrößerung der Fixierungsmittel stattfindet, die deren Fixierungswirkung verbessern kann. Eine weitere Ausführung jener Ausprägung des Erfindungsgedankens sieht vor, dass das oder die Schaumstoff- bzw. Vliesfixierungsmittel formschlüssig mit dem Leitungskörper verbunden, also dort etwa einfach aufgeclipst oder mit einer anderen Rastverbindung festgehalten ist bzw. sind. Alternativ kommt auch eine Klebeverbindung in Betracht.

Neben den Fixierungsmitteln bzw. der Ausgestaltung ihrer Verbindung mit dem Leitungskörper sind auch Mittel zur zeitweiligen Deaktivierung der Verbindungsmittel Gegenstand verschiedener möglicher Ausgestaltungen der Erfindung. So sind bei der erfindungsgemäßen Katheter- oder Elektrodenleitung zweckmäßigerweise steuerbare Deaktivierungsmittel vorgesehen, die die Fixierungsmittel während einer Implantation unwirksam halten, nach korrekter Platzierung des Leitungsendes aber dessen umgehende Fixierung ermöglichen. Hierbei sind die Deaktivierungsmittel von einem proximalen Ende der Leitung her durch den Operateur steuerbar, um die Fixierungsmittel zu aktivieren. In weiter bevorzugter Ausführung weisen die Deaktivierungsmittel eine in Längsrichtung des Leitungskörpers verschiebbare und/oder um eine Längsachse des Leitungskörpers drehbare Schutzhülle auf, die optional mit eingearbeiteten Ausnehmungen zur Unterbringung der Fixierungsmittel im inaktiven Zustand versehen sein kann.

Für den klinischen Einsatz ist eine solche Ausführung der Erfindung wesentlich, dass der Verankerungszustand der Katheter- bzw. Elektrodenleitung von außerhalb des Körpers leicht erfassbar ist. Hierzu ist insbesondere vorgesehen, dass der Leitungskörper und/oder die Fixierungsmittel und/oder das Verbindungselement einen Röntgenmarker aufweisen, der derart ausgebildet ist, dass mit einem Röntgenbild der implantierten Leitung der Aktivierungszustand der Fixierungsmittel von außen erfassbar ist. Der Röntgenmarker-Einsatz bei Kathetern oder Elektrodenleitungen sind seit langem bekannt, und diese können im Rahmen der Erfindung angewandt werden, etwa als metallische Ringe zur Markierung der Verbindungsstelle zwischen Fixierungsmitteln und Leitungskörper, durch metallische Ausführung der Fixierungsmittel selbst, durch eine metallische Markierung der weiter oben erwähnten Schutzhülle, welche die Fixierungsmittel während der Implantation inaktiv hält, etc.

In einer weiteren Ausführung der Erfindung sind die Fixierungsmittel beabstandet vom distalen Ende der Leitung angeordnet. Hiermit wird es beispielsweise möglich, eine mit ihrem distalen Ende in ein Gefäß hineinragende Elektrodenleitung gleichwohl an einem Eingang oder einer Wandung des Gefäßes, nicht aber im Gefäß selbst mittels der Fixierungsmittel zu verankern und somit eine Störung des Blutstromes durch die Fixierungsmittel oder ein unerwünschtes Zuwuchern des Gefäßes durch um die Fixierungsmittel herumwachsendes Gewebe zu verhindern.

In einer weiteren Ausführung der Erfindung ist vorgesehen, dass die Fixierungsmittel ein vom Leitungskörper abspreizbares oder bei Aktivierung der Fixierungsmittel selbst-abspreizendes Spreizelement aufweisen. Es kann sich hierbei tatsächlich um ein einzelnes Ankerartiges Element handeln, aus derzeitiger Sicht bevorzugt ist aber eine Ausführung, bei der die Fixierungsmittel eine Gruppe von Spreizelementen aufweisen, die insbesondere um den Umfang des Leitungskörpers verteilt angeordnet sind und diesen im aktivierten Zustand insbesondere trichter-, kelch- oder tulpenförmig umgeben. Eine solche Gruppe von Fixierungsmitteln bietet in der Regel einen sichereren Halt für die Elektrodenleitung bis zum Einwachsen und stellt auch weniger hohe Anforderungen an die Geschicklichkeit des Operateurs während der Implantation.

Eine weitere zweckmäßige Ausführung der Erfindung sieht vor, dass das selbstabspreizende Spreizelement oder die Gruppe von Spreizelementen als elastisch in den aktiven Zustand vorgespanntes Metallteil, insbesondere aus Nitinol oder einer ähnlich wirksamen Metalllegierung, ausgeführt ist bzw. sind. Des Weiteren ist es sinnvoll, dass das oder jedes Spreizelement zur Reduzierung einer Traumatisierung einer Körperhöhlungs- bzw. Gefäßwand bei der Fixierung spaten-, teller-, schlangen-, spiral- oder schraubenförmig ausgebildet sind oder entsprechend ausgebildete Enden hat.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1: eine erste Ansicht einer Stimulationselektrodenleitung gemäß einer Ausführung der Erfindung (mit deaktivierten Fixierungsmitteln),
- Fig. 2: eine weitere Ansicht der Elektrodenleitung nach Fig. 1 (mit aktivierten Fixierungsmitteln),
- Fig. 3a bis 3h: Ansichten verschiedener Ausführungen von Fixierungsmitteln der Stimulationselektrodenleitung,
- Fig. 4: eine skizzenartige Darstellung des Zusammenwirkens von Fixierungsmitteln und einem zerstörbaren Haltering bei einer weiteren Ausführung der erfindungsgemäßen Katheter- bzw. Elektrodenleitung,
- Fig. 5a und 5b: zwei Detailansichten spezieller Ausführungen des Verbindungsbereiches zwischen Fixierungsmitteln und Leitungskörper und
- Fig. 6a bis 6f: schematische Darstellungen von Ausführungen der Elektrodenleitungen gemäß der zweiten Ausprägung des Erfindungsgedankens.
- Fig 7a bis 7c: zeigt schematisch den aktiven und inaktiven Zustand der Fixierungselemente und die Lage von röntgensichtbaren Markierungen

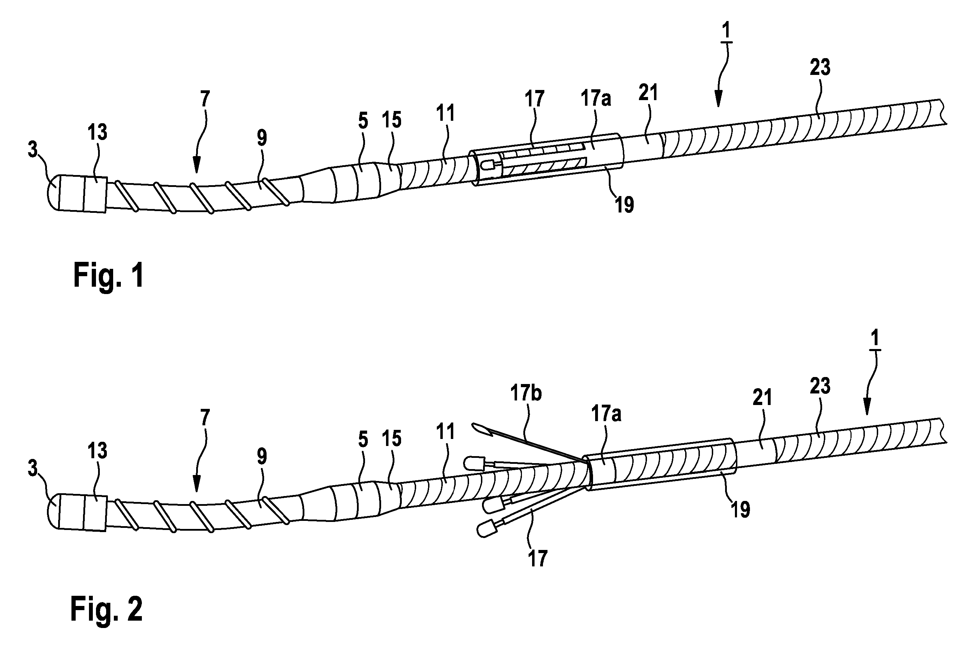

Fig. 1 und 2 zeigen den distalen Teil einer Schrittmacherelektrodenleitung 1 mit einer am distalen Ende angeordneten Tip-Elektrode 3 und einer hiervon beabstandet angeordneten Ring-Elektrode 5 auf einem flexiblen Kunststoff-Leitungskörper 7, der beispielsweise durch einen Silikonschlauch mit die Gleiteigenschaften verbessernder Parylene-Beschichtung gebildet sein kann. Innerhalb des Leitungskörpers 7 verlaufen gewendelte Elektrodenleitungen 9 und 11, und am Ort der Tip-Elektrode 3 und der Ring-Elektrode 5 ist jeweils ein Röntgenmarker 13 bzw. 15 vorgesehen. In einer bevorzugten Ausführungsform können die Elektroden 3 und 5 als Röntgenmarker ausgebildet sein.

Auf den Leitungskörper 7 ist ein Spreizelement 17 aus Nitinol aufgeklemmt, das in dem in Fig. 1 gezeigten inaktiven Zustand durch eine vom proximalen Ende der Elektrodenleitung 1 aus darüber geschobene Schlauchhülle 19 mit einem Platin-Anschlagring 21 gehalten wird. Diese sind am vorderen (distalen) Ende eines innen gleitfähig beschichteten PU-Schlauches 23 angebracht. In diesem Zustand kann die Schrittmacherelektrodenleitung 1 widerstandsarm in den Körper eines Patienten eingeschoben werden.

In dem in Fig. 2 gezeigten Zustand ist der PU-Schlauch 23 mit der darin befestigten Schlauchhülle 19 etwas zurückgezogen, und das Spreizelement 17 hat sich aufgespreizt und ist nunmehr im für eine Fixierung der Elektrodenleitung 1 aktiven Zustand. In der in Fig. 1 und 2 gezeigten Ausführung umfasst es vier von einem Platin-Klemmring 17a sich trichterförmig abspreizende Arme 17b deren Enden zur Reduzierung einer Traumatisierung des von ihnen kontaktierten Körpergewebes verbreitert spatenförmig ausgebildet sind. Das Spreizelement kann temporär mit der Elektrode verbunden sein. Nach der Zerstörung der Verbindung löst sich das Spreizelement vom Leitungskörper und wird somit unwirksam. Nach dem Unwirksamwerden der Fixierung wird der Leitungskörper nur noch durch Gewebe gehalten, welches ihn zwischenzeitlich umwachsen hat, und die Elektrodenleitung 1 kann daher im Bedarfsfall wieder explantiert werden, ohne das es erhebliche Traumatisierungen des umgebenden Körpergewebes gäbe.

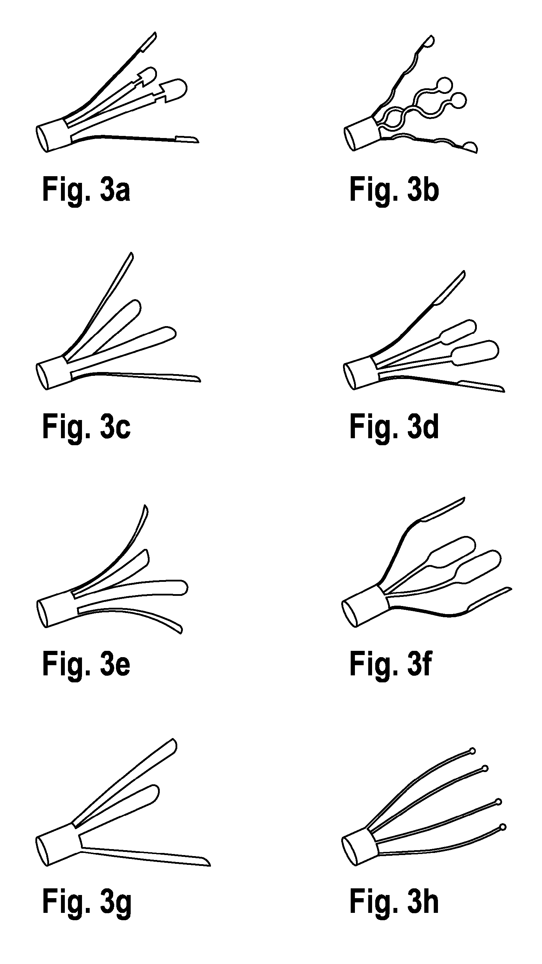

In den Figuren 3a bis 3h sind verschiedene Ausführungen von Spreizelementen bzw. Fixierungsmitteln in Art des Spreizelementes 17 aus Fig. 1 und 2 dargestellt. Diese Darstellungen sind im Wesentlichen selbsterklärend, so dass sich eine zusätzliche verbale Beschreibung erübrigt. Es sei lediglich darauf hingewiesen, dass die Mehrzahl der einzelnen Spreizarme der gezeigten Spreizelemente verschiedene Umfangsformen beschreiben kann, neben Trichterform (Fig. 3a bis 3d und 3g) auch eine Kelchform (Fig. 3e) oder Tulpenformen (Fig. 3f und 3h). Unter mehreren kommerziell erhältlichen Ausführungen lässt sich für einen Operateur ohne Weiteres eine Auswahl treffen, die an den konkreten Einsatzort einer zu implantierenden Katheter- oder Elektrodenleitung im Hinblick auf eine sichere und gleichwohl möglichst wenig traumatisierende Fixierung optimal angepasst ist.

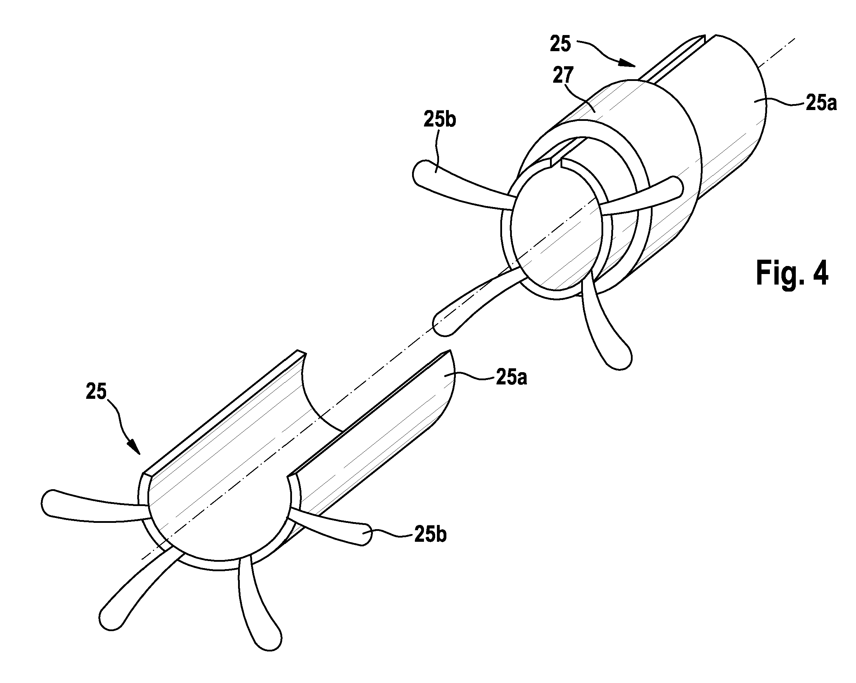

Das Wirkprinzip einer bevorzugten Ausführung der Erfindung wird anhand von Fig. 4 besonders deutlich. Die Figur zeigt ein federelastisch deformierbares Fixierungselement 25 mit einem Ringabschnitt 25a und vier Spreizarmen 25b einerseits (links unten) im entspannten Zustand, den das Element vor einem Aufklemmen auf einen Leitungskörper und nach einem Lösen von diesem einnimmt, und anderseits (rechts oben) in einem ringförmig geschlossenen Zustand, in dem es auf einem - nicht dargestellten - Leitungskörper durch einen Haltering 27 gehalten wird. In diesem Beispiel kann das Fixierelement beispielsweise aus Nitinol oder einer ähnlich wirksamen Metalllegierung hergestellt sein. Denkbar wäre als Material für das Fixierelement auch ein Kunststoff mit federelastischen Eigenschaften.

Der Ringabschnitt oder Bund 25a des Fixierungselementes 25 ist also im entspannten Zustand weit geöffnet und wird bei der Montage auf einem Leitungskörper geschlossen, womit er unter Spannung kommt. In diesem Zustand wird der Haltering 27 über den Bund 25a geschoben, so dass das Fixierungselement sich nicht wieder öffnen kann. Da der Ring aus einem resorbierbaren Material gefertigt ist, kann sich nach dessen Abbau das Fixierungselement wieder vom Leitungskörper lösen, indem es in seine ursprüngliche, annähernd halbkreisförmige, offene Form zurückfedert.

Als resorbierbare Materialien für den Haltering sind etwa Polylactide oder andere körperverträgliche Kunststoffe oder Magnesium oder ein anderes körperverträgliches und resorbier- bzw. zersetzbares Metall geeignet, grundsätzlich kommen auch resorbierbare Fäden bzw. Nahtmaterialien für diesen Zweck in Frage. Bei bestimmten Materialien - etwa Magnesium - kommt eine gezielte elektrochemische Auflösung durch eine im Hinblick auf die Spannungs- bzw. Stromempfindlichkeit der angrenzenden Körperorgane geeignet gewählte Beaufschlagung mit elektrischer Spannung in Betracht.

Eine geeignete Zeitdauer bis zur Resorption bzw. Zersetzung des Halteringes und damit bis zum Lösen der Verbindung zwischen Leitungskörper und Fixierungselement kann über geeignete Materialwahl (etwa Auswahl des Polymerisationsgrades und speziellen Compounds des Polymers oder Auswahl einer geeigneten Metalllegierung) und über die Dimensionen (insbesondere die Wandstärke) des Ringes voreingestellt werden. Nach vorliegenden klinischen Erfahrungen sollte sich die Fixierung nach 2-3 Wochen vom Leitungskörper lösen, denn dann ist die Elektrodenleitung durch umwachsendes Gewebe bereits so gut stabilisiert, dass sie nicht mehr selbsttätig disloziert.

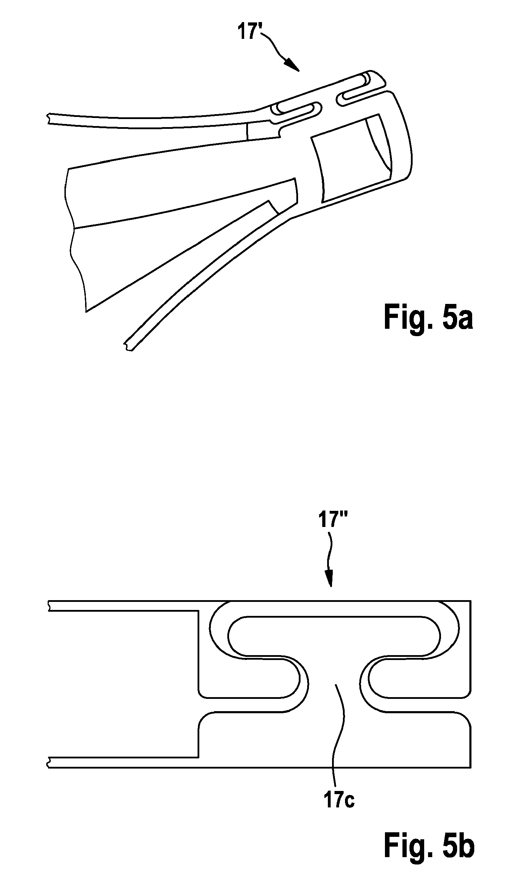

Fig. 5a und 5b zeigen modifizierte Spreizelemente 17' und 17", deren Ringabschnitt zur Befestigung auf einem Leitungskörper federelastisch ausgeführt ist und Ausnehmungen zur Ausbildung eines kombinierten Kraft- und Formschutzes zwischen Spreizelement und Leitungskörper (Fig. 5a) oder eine durch radialen Druck zu schließende und anschließend selbsthemmende Schließe 17c (Fig. 5b) aufweist.

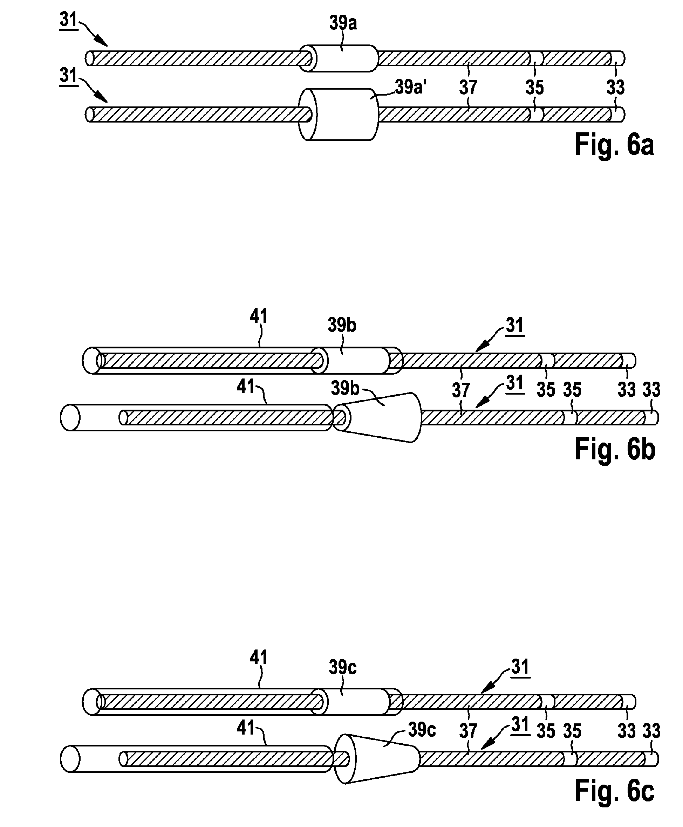

Die Figuren 6a bis 6f zeigen als Prinzipskizzen als weitere Ausführungsformen Elektrodenleitungen mit elastisch deformierbaren Schamstoffelementen, teilweise in ihrem Zustand während der Implantation und nach der Aktivierung. In Anlehnung an die bei der Darstellung und Beschreibung der ersten Ausprägung des Erfindergedankens benutzten Bezugsziffer ist in allen Ausführungen der Fig. 6a bis 6f die Elektrodenleitung mit der Ziffer 31, eine Tipelektrode mit der Ziffer 33, eine Ringelektrode mit der Ziffer 35 und ein flexibler Kunststoff-Leitungskörper mit der Ziffer 37 bezeichnet.

Fig. 6a zeigt zwei Ausführungen der Elektrodenleitung 31 mit jeweils einem in proximaler Richtung beabstandet von der Ring-Elektrode 35 auf dem Leitungskörper 37 vorgesehenen ringförmigen Schaumstoff-Fixierungselement 39a bzw. 39a', wobei sich das letztere Fixierungselement durch seinen größeren Durchmesser vom ersteren unterscheidet.

Bei den Ausführungen der Elektrodenleitung 31 nach Fig. 6b und 6c ist jeweils ein kegelstumpfförmiger Schaumstoff-Fixierungsring 39b (Fig. 6b) bzw. 39c (Fig.6c) vorgesehen, wobei die Ausrichtung des jeweiligen Konus in beiden Ausführungen umgekehrt ist. In beiden Figuren (wie auch in den nachfolgenden) ist jeweils der Zustand während der Implantation der Elektrodenleitung, also mit über das Fixierungselement geschobenem und dieses inaktiv machenden Silikonschlauch 41, bzw. nach der Implantation, also mit zurückgezogenem Silikonschlauch und voll expandiertem und somit aktivem Fixierungselement, dargestellt.

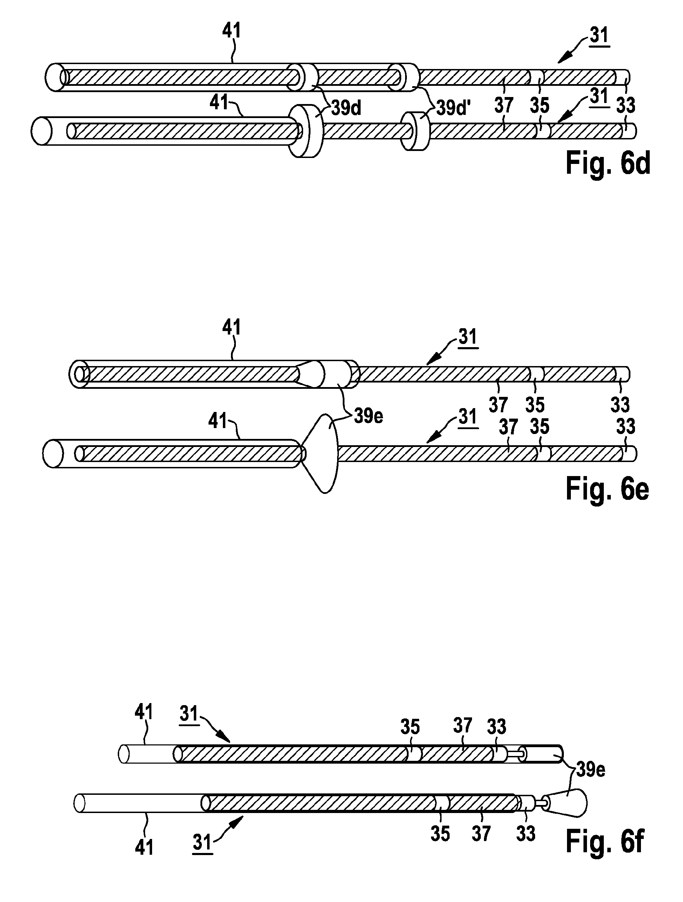

Fig. 6d zeigt als weitere Ausführung eine Elektrodenleitung mit zwei von einander beabstandeten Fixierungsringen 39d und 39d' mit unterschiedlichem Durchmesser und relativ geringer Höhe, die beide proximal von der Ringelek-trode 35 platziert sind.

Fig. 6e zeigt als weitere Modifikation ein pilzkopfartiges bzw. haubenförmiges Schaumstoff-Fixierungselement 39e, welches, ebenso wie bei den weiter oben beschriebenen Ausführungen, proximal von der Ringelektrode 35 an der Elektrodenleitung (bevorzugt formschlüssig) angebracht ist. Fig. 6f schließlich zeigt eine hinsichtlich der Platzierung des Fixierungsmittels grundsätzlich andere Ausführung: Hier befindet sich ein kegelstumpfförmiges Fixierungselement 39f, dessen Durchmesser sich zu einem entspannten und somit aktivierten Zustand aufweitet, am distalen Ende der Elektrodenleitung 31, also noch jenseits der Tip-Elektrode 33.

Die Schaumstoff- oder Vlieselemente können in ihren Maßen und in ihrer Form variieren: sie können z. B. konisch, oval, quadratisch, kugelförmig u. a. geformt sein. Der Schaumstoff und alle an der Fixierung beteiligten Teile sind resorbierbar z. B. aus Polyurethan, Polylaktat, Polyglykol (Polyethylenglykol), Gelatine, Kollagen, oder aus Tamponaden die zur Blutstillung angewendet werden wie z. B. Gelaspon®. Die Fixierung wird in einer definierten Zeit, die je nach Schaumstoffart, -dichte oder -dicke eingestellt werden kann, vollständig resorbiert. Der Schaumstoff oder das Vlies kann mit gerinnungsbeeinflussenden Mitteln oder anderen Wirkstoffen versehen werden.

Für den Implantations- wie auch einen etwaigen Explantationsvorgang ist eine Beobachtung von außerhalb des Körpers über ein Röntgenbild wünschenswert.

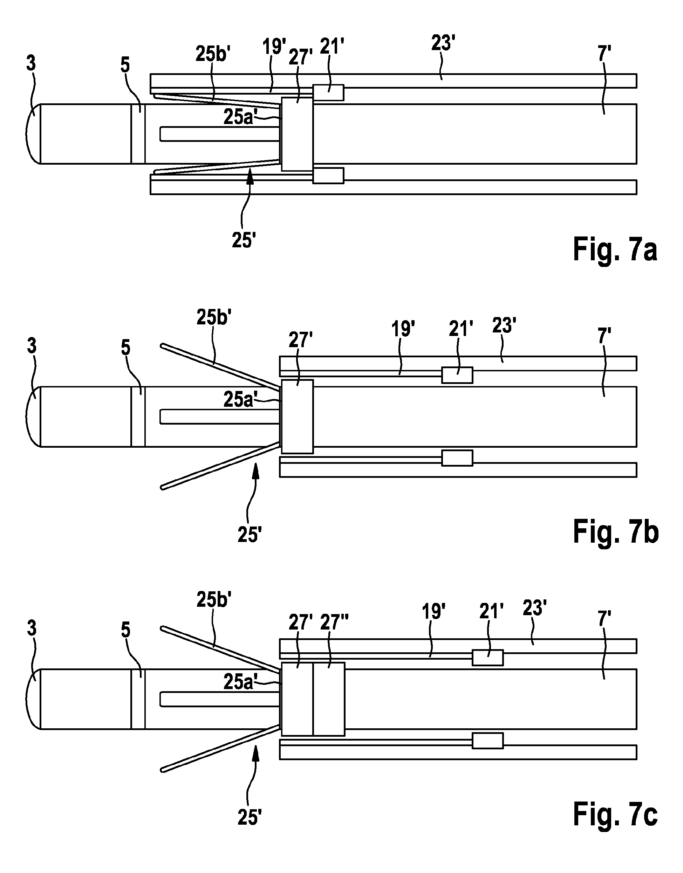

Fig. 7a bis 7c zeigen beispielhaft die Lage von Röntgenmarkern, mit Hilfe derer ein Operateur erkennen kann, ob die Fixierungsmittel inaktiv oder aktiv sind. So zeigt Fig. 7a beispielhaft eine Elektrodenleitung mit röntgensichtbaren Tip- und Ringelektroden 3 und 5 sowie einem Fixierungselement 17. Das Fixierungselement ist nicht röntgensichtbar. Das Fixierungselement 25' durch einen Haltering 27' auf der Elektrodenleitung fixiert. Der Haltering 27' auf dem Klemmring 25a' ist durch die Wahl eines geeigneten Materials röntgensichtbar ausgelegt. In einer weiteren Ausgestaltung ist der Haltering 27' biodegradierbar ausgestaltet, beispielsweise wie weiter oben beschrieben aus einem biodegradierbaren Metall oder Kunststoff. In diesem Fall wird die Röntgensichtbarkeit durch Zumengung von röntgensichtbaren Materialien wie beispielsweise Goldstaub hergestellt. In einer in Fig. 7c gezeigten Ausgestaltung kann zusätzlich zu einem biodegradierbaren Haltering 27' - der in diesem Falle nicht röntgensichtbar sein muss - ein Markerring 27" vorgesehen werden. Der Markerring bedeckt dann nicht den Klemmring 25a' des Fixierungselementes.

In Fig. 7a ist wie unter der Beschreibung zu Fig. 1 beschrieben, die Elektrodenleitung in einem Zustand, indem sie in den Körper implantiert werden kann, das heißt, dass die am PU-Schlauch 23' befestigte Schlauchhülle 19' das Fixierungselement inaktiv hält. Der Anschlagring 21' der die Schlauchhülle 19' gegen den PU-Schlauch 23' abgrenzt und diesen befestigt, ist in diesem Ausführungsbeispiel aus einem röntgensichtbaren Material hergestellt, beispielsweise aus Gold oder Platin.

Damit der Operateur nun erkennen kann, ob sich die Fixierung in einem aktiven, in dem Gefäß befestigten, oder inaktiven Zustand befindet, kann er sich mit Hilfe eines Röntgenbildes oder der Fluoroskopie bedienen. Dabei ist die Lage der Tip-Elektrode 3 und der Ring-Elektrode 5 kennzeichnenden Röntgenmarker (Metallringe) 13 und 15 bzw. die selbst röntgensichtbaren Elektroden 3 und 5 einerseits als auch die relative Lage des röntgensichtbaren Halteringes 27' und des Anschlagringes 21' von Bedeutung.

Beim Einführen der Elektrodenleitung, wenn die Elektrode an den Implantationsort verbracht wird, und bei Inaktivstellung des Fixierungselementes 25' stößt der Anschlagring 21' unmittelbar an den Haltering 27'. Nimmt der Operateur ein Röntgenbild zu Hilfe, erkennt er im Röntgenbild drei deutliche Markersignale: von der Tip- und der Ringelektrode 3 und 5 (oder den zugeordneten Röntgenmarker 13 und 15) und ein Signal von den unmittelbar aneinander liegenden Haltering 27' und Anschlagring 21'. Dieser Zustand ist in Fig. 7a dargestellt.

Am Implantationsort kann der Operateur das Fixierungselement 25' aktivieren und dadurch die Elektrode mittels der abspreizbaren Arme 25b' befestigen, indem er den PU-Schlauch 23' in die proximale Richtung zurückzieht. Durch diese Bewegung bewegt sich der röntgensichtbare Anschlagring 21' vom röntgensichtbaren Haltering 27' in proximale Richtung weg. Dadurch werden nun im Röntgenbild vier deutliche Markersignale sichtbar: zwei Signale für die Elektroden 2 und 5 (oder den zugeordneten Röntgenmarker 13 und 15) und je ein Signal für den Haltering 27' und den Anschlagring 21'. Dies zeigt dem Operateur an, dass das Fixierungselement 25' freigesetzt ist. Dieser Zustand ist in Fig. 7b dargestellt.

Fig. 7c zeigt den Zustand des aktivierten Fixierungselementes 25' mit einem separaten Markerring 27" dar, wobei auch hier vier Markersignale in einem Röntgenbild erkennbar sind.

Bei einer Fehlpositionierung der Elektrodenleitung ist auch eine Umpositionierung möglich, indem der PU-Schlauch 23' wieder so in distaler Richtung verschoben wird, dass das Fixierungselement 25' inaktiv wird. Die Inaktivstellung, in der der Arzt ohne Verletzungsrisiko für den Patienten die Elektrodenleitung umpositionieren kann, erkennt der Operateur am Umstand, dass im Röntgenbild wieder nur drei Markersignale erkennbar sind.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Katheter- oder Elektrodenleitung (1) mit einem langgestreckten biegsamen Leitungskörper (7, 7') und mit dem Leitungskörper verbundenen Fixierungsmitteln (17, 17', 17", 25, 25') zur Fixierung der Katheter- oder Elektrodenleitung in vorbestimmter Lage in einem Gefäß oder einer Körperhöhlung eines Patienten, wobei zwischen dem Leitungskörper und den Fixierungsmitteln eine lösbare Verbindung vorgesehen ist derart, dass nach Lösen der Verbindung eine Explantation der Katheter- oder Elektrodenleitung unter Verbleib der Fixierungsmittel im Körper des Patienten möglich ist.

2. Katheter- oder Elektrodenleitung nach Anspruch 1, wobei die Verbindung ein resorbierbares oder sich selbsttätig zersetzendes Verbindungselement (27, 27') aufweist.

3. Katheter- oder Elektrodenleitung nach Anspruch 1, wobei die Verbindung ein durch Energieeintrag in die Leitung, insbesondere durch Anlegen eines elektrischen Stromes oder Einleitung von UV-Strahlung, Licht oder IR-Strahlung, zerstörbares Verbindungselement aufweist.

4. Katheter- oder Elektrodenleitung nach einem der Ansprüche 2 oder 3, wobei die Verbindung einen resorbier- oder zerstörbaren Haltering (27, 27') aufweist, der die Fixierungsmittel (17, 17', 25, 25') nur in seinem intakten Ausgangszustand auf dem Leitungskörper festhält.

5. Katheter- oder Elektrodenleitung nach einem der Ansprüche 2 bis 4, wobei die Fixierungsmittel auf separaten Ringsegmenten (17a, 25a, 25a') angeordnet sind, die den Leitungskörper (7, 7') jeweils nur abschnittsweise umschließen und vom Verbindungselement (27, 27') auf dem Leitungskörper festgehalten werden, nach Resorption oder Zerstörung des Verbindungselementes sich aber vom Leitungskörper lösen.

6. Katheter- oder Elektrodenleitung nach einem der vorangehenden Ansprüche, wobei der Leitungskörper (7, 7') und/oder das Verbindungselement (27') einen Röntgenmarker aufweisen, der derart ausgebildet ist, dass mit einem Röntgenbild der implantierten Leitung der Aktivierungszustand der Fixierungsmittel (25') von außen erfassbar ist.

7. Katheter- oder Elektrodenleitung nach einem der vorangehenden Ansprüche, wobei die Fixierungsmittel (17, 17', 17", 25, 25') ein vom Leitungskörper (7, 7') abspreizbares oder bei Aktivierung der Fixierungsmittel selbst-abspreizendes Spreizelement (17b, 25b, 25b') aufweisen.

8. Katheter- oder Elektrodenleitung nach Anspruch 7, wobei die Fixierungsmittel (17, 17', 17", 25, 25') eine Gruppe von Spreizelementen (17b, 25b, 25b') aufweisen, die insbesondere um den Umfang des Leitungskörpers verteilt angeordnet sind und diesen im aktivierten Zustand insbesondere trichter-, kelch- oder tulpenförmig umgeben.

9. Katheter- oder Elektrodenleitung nach Anspruch 7 oder 8, wobei das selbstabspreizende Spreizelement oder die optionale Gruppe von Spreizelementen (17b, 25b, 25b') als elastisch in den aktiven Zustand vorgespanntes Metallteil, insbesondere aus Nitinol oder einer ähnlich wirksamen Metalllegierung oder Kunststoff, ausgeführt ist bzw. sind.

10. Implantierbare Katheter- oder Elektrodenleitung (31) mit einem lang gestreckten biegsamen Leitungskörper (37) und mit dem Leitungskörper verbundenen Fixierungsmitteln (39a - 39f) zur Fixierung der Katheter- oder Elektrodenleitung in vorbestimmter Lage in einem Gefäß oder einer Körperhöhlung eines Patienten, wobei die Fixierungsmittel ein resorbierbares formelastisches Schaumstoff- oder Vlieselement aufweisen.

11. Katheter- oder Elektrodenleitung nach Anspruch 10, wobei das oder jedes Schaumstoff- oder Vlieselement (39a - 39f) ein in Blut quellfähiges Material und/oder ein gerinnungshemmendes Mittel aufweist.

12. Katheter- oder Elektrodenleitung nach Anspruch 11 oder 12, wobei das oder jedes Schaumstoff- oder Vlieselement (39a - 39f) formschlüssig mit dem Leitungskörper (37) verbunden ist.

13. Katheter- oder Elektrodenleitung nach Anspruch 2, wobei Material und Dimensionen des Verbindungselementes (39a - 39f) bzw. der resorbierbaren Fixierungsmittel derart gewählt sind, dass die Resorption oder Zersetzung nach einer vorbestimmten Zeitspanne zum Lösen der Verbindung bzw. zum Auflösen der Fixierungsmittel führt, innerhalb derer die Katheter- oder Elektrodenleitung (31) in einem solchen Maße von Körpergewebe umwachsen ist, dass sie nicht mehr selbsttätig disloziert.

14. Katheter- oder Elektrodenleitung nach einem der vorangehenden Ansprüche, wobei steuerbare Deaktivierungsmittel (23, 23', 41) vorgesehen sind, die die Fixierungsmittel (17, 17', 17", 25, 25', 39a - 39f) während einer Implantation unwirksam halten.

15. Katheter- oder Elektrodenleitung nach Anspruch 14, wobei die Deaktivierungsmittel (23, 23', 41) eine in Längsrichtung des Leitungskörpers verschiebbare und/oder um eine Längsachse des Leitungskörpers drehbare Schutzhülle (19, 19'), optional mit eingearbeiteten Ausnehmungen, aufweisen.
